# EUROPEAN PATENT SPECIFICATION

(11) **EP 0 438 470 B1**
(45) Date of publication and mention of the grant of the patent: **01.06.1994**
(21) Application number: 89911557.0
(22) Date of filing: 09.10.1989
(51) Int. Cl.: B01L 3/00, G01N 33/543

(54) **A sample plate with a plurality of sample wells intended for radiolabelled binding assays**
Probenplatte mit einer Vielzahl von Probengefässen für radiomarkierte Bindetestverfahren
Plaque porte-échantillons avec plusieurs cuvettes a échantillons destinée à des tests de liaison radiomarqués

(30) Priority: 11.10.1988 SE 8803602
(43) Date of publication of application: 31.07.1991
(73) Proprietor: WALLAC OY, SF-20101 Turku (FI)
(72) Inventor: OIKARI, Timo, SF-20500 Turku (FI); YRJÖNEN, Tapio, SF-20740 Turku (FI); LEHTINEN, Kauko, SF-21260 Raisio (FI)
(74) Representative: Berg, Sven Anders
(86) International application number: FI8900191
(87) International publication number: WO9003844

(56) References cited:
- WO-A-88/04429
- US-A- 3 646 346
- US-A- 4 568 649

## Description

A common technique in chemistry and biosciences is to coat suitable solid phases, such as microparticles or inner walls of test tubes, with a compound that can specifically bind a reactant in solution being investigated. Often, the mentioned reactant is radioactively labelled and gets bound onto the solid phase while the binding reaction proceeds. The amount of bound (sometimes unbound) radioactivity is then measured. This requires a procedure to separate the unbound fraction (in solution) from the bound fraction (on the solid phase).

A method to avoid the separation step is presented in US-A-4,568,649. In this patent a plurality of small plastic support particles or beads (diameter about one micrometer) impregnated with a fluorescer are coated appropriately, and the beads are added into the solution containing the radiolabelled reactant.

Some common radiolabels (e.g. H-3 and I-125) emit low-energy electrons with short ranges (some micrometers) in water solutions and only the bound label gets close enough to the plastic so that the electrons can reach it and excite the fluorescer with subsequent light emission, the scintillation. The light is then detected with a suitable detector, such as a photomultiplier device.

However, the addition of beads is an extra step and there may be problems with buoyancy if the densities of the beads and the solution do not match. Furthermore, in some cases some loose nonspecific binding can occur requiring separation which is difficult to perform with beads. The present invention overcomes these drawbacks.

Besides of single tubes or cuvettes, multi-well sample plates which comprise several separate sample wells have become widespread test plates for in vitro analyses. The plates often have 96 wells arranged in eight rows and twelve columns, and the volume of each well is 200-300 micro-liters. The applicants have found that it is possible to prepare tubes, cuvettes or multi-well plates of plastic scintillator and to coat them with a binding compound enabling non-separation radiolabel binding assays with considerable simpleness.

Figure 1 shows a vertical section of a part of a sample plate which has several separate sample wells produced from a transparent plastic scintillator.

Figure 2 shows a vertical section of a part of a sample plate which has several separate sample wells produced from a two-layer transparent plastic sheet.

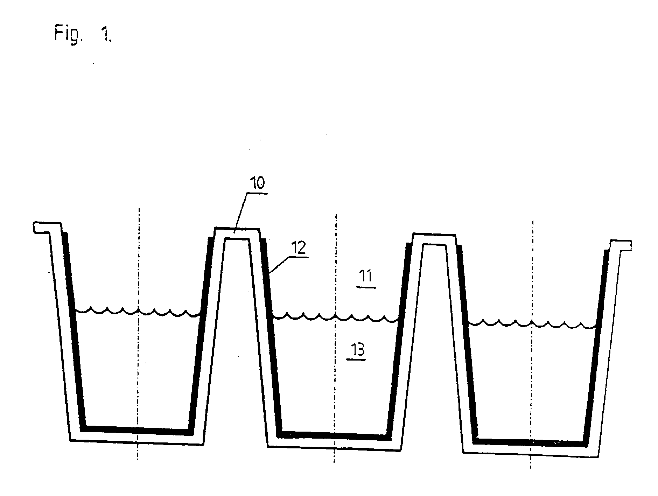

Figure 1 shows a sample plate (10) with a plurality of sample wells (11) intended for radiolabelled binding assays. Said sample plate (10) can be produced from transparent scintillation plastic for example by a vacuum thermoforming or by an injection moulding process.

Said sample well (11) contains a test sample solution (13) including the radiolabelled reactant. The inner wall of said sample well (11) is coated with a binding compound (12) that specifically binds to the reactant being investigated.

The portion of said radiolabelled reactant that binds to said binding compound surface (12) is close enough to said wall of the sample well (11) so that radiation emitted by said bound radiolabelled reactant can interact with said plastic scintillator which converts a part of the absorbed energy into light which can be measured by a proper apparatus which is not shown here.

The radiation emitted by said radiolabelled reactant that does not bind to said binding compound coated surface (12) of said sample well (11) cannot interact with the plastic scintillator material of said sample well (11) because the distance of this portion of said radiolabelled reactant from the walls of said sample wells (11) normally exceeds the range of the radiation emitted by said radiolabelled reactant. Typically said reactant is labelled with low energy beta particles emitting isotope such as tritium (H-3). The maximum range of the beta particles emitted by tritium is only a few micrometers in said test sample solution (13).

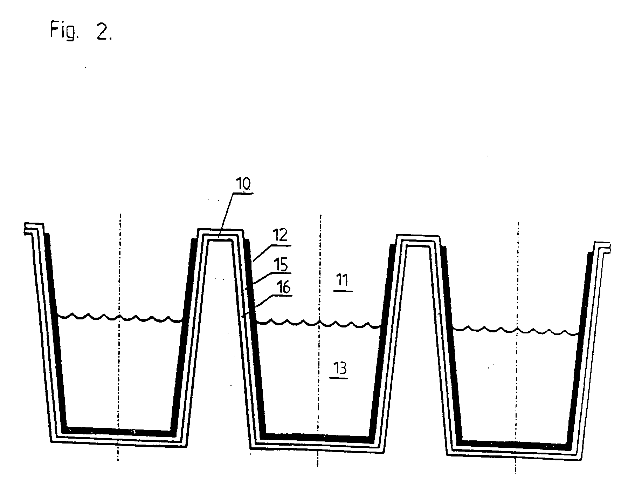

Figure 2 shows an alternative construction of a sample plate (10) with a plurality of sample wells (11) intended for radiolabeled binding assays. In this case said sample plate (10) is produced from a two-layer transparent plastic sheet, where the inner layer (scintillation layer) (15) of said sample wells (11) is plastic scintillator and the outer layer (support layer) (16) is some suitable transparent plastic. This kind of plate can be produced by a vacuum thermoforming process.

The invention is not confined to the above embodiments alone, but it may show even considerable variation within the scope of the patent claims.

Plastic scintillators are well known for those skilled in the art. They are non-fluid solutions consisting of fluorescent organic additives, called fluors, dissolved in solidified polymer. The best polymers are aromatic polystyrene and polyvinyltoluene and a common primary fluor is diphenyloxazole (PPO). Often a secondary fluor, such as 1,4-bis-2-(5-phenyloxazolyl)-benzene (POPOP), is added to obtain better match with the photomultiplier spectral response.

## Claims

1. A sample plate with a plurality of sample wells intended for radiolabelled binding assays, characterized in that said sample plate or said wells of said sample plate are produced from transparent scintillation plastic by a vacuum thermoforming or by an injection moulding process.

2. A sample plate according to claim 1, characterized in that said sample plate (10) is produced from a two-layer transparent plastic sheet, where the inner layer (scintillation layer) (15) of said sample wells (11) is of transparent scintillation plastic and the outer layer (support layer) (16) is of some suitable transparent plastic.

3. A sample plate according to claims 1 and 2, characterized in that the inner surfaces of said sample wells of said sample plate are coated with a binding compound (12) that specifically binds to the radiolabelled reactant being investigated.

## Patentansprüche

1. Probenplatte mit einer Mehrzahl von Probenvertiefungen für radioaktiv markierte Bindungstests, dadurch gekennzeichnet,
daß die Probenplatte oder die Vertiefungen der Probenplatte aus einem durchsichtigen Szintillationskunststoff durch Vakuumtiefziehen oder durch Spritzformen hergestellt ist (sind).

2. Probenplatte nach Anspruch 1, dadurch gekennzeichnet,
daß die Probenplatte (10) aus einer zweilagigen durchsichtigen Kunststoffolie hergestellt ist, wobei die innere Schicht (Szintillationsschicht) (15) der Probenvertiefungen (11) aus einem durchsichtigen Szintillationskunststoff und die äußere Schicht (Trägerschicht) (16) aus irgendeinem geeigneten durchsichtigen Kunststoff bestehen.

3. Probenplatte nach Ansprüchen 1 und 2, dadurch gekennzeichnet,
daß die Innenflächen der Probenvertiefungen der Probenplatte mit einer Bindungsverbindung (12), die eine spezifische Bindung mit dem zu untersuchenden radioaktiv markierten Reaktionsteilnehmer eingeht, beschichtet sind.

## Revendications

1. Une plaque porte-échantillons avec plusieurs cuvettes à échantillon destinée à des tests de liaison radiomarqués, caractérisée par le fait que ladite plaque porte-échantillons ou lesdites cuvettes de ladite plaque porte-échantillons sont produites à partir d'une matière plastique transparente à scintillation par un procédé de thermoformage sous vide ou par un procédé de moulage par injection.

2. Une plaque porte-échantillons suivant la revendication 1, caractérisée par le fait que ladite plaque porte-échantillons (10) est produite à partir d'une feuille de plastique transparente à deux phases, dans laquelle la phase interne (phase à scintillation) (15) desdites cuvettes à échantillon (11) est en matière plastique transparente à scintillation et la phase externe (phase support) (16) est constituée d'une matière plastique transparente appropriée.

3. Une plaque porte-échantillons suivant les revendications 1 et 2, caractérisée par le fait que les surfaces internes desdites cuvettes à échantillon de ladite plaque porte-échantillons sont enduites avec un composé liant (12) qui se lie spécifiquement avec le réactif radiomarqué étudié.
